# EUROPEAN PATENT SPECIFICATION

(11) **EP 1 635 746 B1**
(45) Date of publication and mention of the grant of the patent: **28.05.2008**
(21) Application number: 04739813.6
(22) Date of filing: 11.06.2004
(51) Int. Cl.: A61F 2/46

(54) **MIXING AND DISTRIBUTION DEVICE FOR FIXING PASTE, PARTICULARLY FOR MULTICOMPONENT BONE CEMENT**
MISCH- UND VERTEILVORRICHTUNG FÜR FIXIERPASTE, INSBESONDERE FÜR MEHRKOMPONENTEN-KNOCHENZEMENT
DISPOSITIF DE MELANGE ET DE DISTRIBUTION POUR PATE DE FIXATION, NOTAMMENT POUR OS-CIMENT A COMPOSANTS MULTIPLES

(30) Priority: 25.06.2003 IT PD20030139
(43) Date of publication of application: 22.03.2006
(73) Proprietor: Bidoia s.a.s. di Gianfranco Bidoia E C., I-35010 Vigonza (Padova) (IT)
(72) Inventor: BIDOIA, Gianfranco, I-35142 Padova (IT)
(74) Representative: Modiano, Micaela Nadia
(86) International application number: PCT/EP2004/006323
(87) International publication number: WO 2005/000171

(56) References cited:
- US-A- 4 277 184
- US-A- 5 842 786
- US-A1- 2002 092 871

## Description

### Technical Field

The present invention relates to a mixing and distribution device for fixing paste, particularly for multicomponent bone cement.

### Background Art

Adhesives, adhesive pastes and fixing pastes in general are currently used in many fields to mutually fix two parts. These products are often produced by mixing two different components, which form the final product by reacting.

The use of these products can cause several problems, such as for example the release of toxic gases during mixing, rapid drying of the product once it has formed, difficulty in applying it to the parts to be bonded, et cetera.

One of the fields in which the use of this product is particularly delicate is orthopedic surgery.

In this field it is in fact necessary to replace certain bone components, such as for example the head of the femur and/or the cotyloid cavity that engages the head in the pelvis, with artificial prostheses made of the most disparate materials, especially metallic ones, but also plastic materials or materials derived from ceramic (for example porcelain).

Alongside the problem of replacement, there is the fundamental need to fix said artificial prostheses as rigidly as possible to the reference bone.

Substantially two approaches are used to achieve this fixation.

The first approach implies coupling the prosthesis to the reference bone by way of mechanical connections such as screws, cerclages, et cetera, while the second approach, which is the one most widely used at present, provides for adhesive fixing elements.

This second approach is always used in any case when, after a certain time, the mechanical connections fail without the possibility of restoring them or replacing them with other mechanical fixations.

As regards this second approach, an acrylic-based cement is predominantly used at present.

The acrylic cement currently used is obtained from the combination of two components: one in powder form (usually a polymethyl methacrylate) and one in liquid form (usually a methyl or butyl methacrylate or a combination thereof), in specific proportions, which are placed in mutual contact.

The preparation and subsequent use of this cement, however, has drawbacks.

Combining these two components in fact currently entails some problems in use.

First of all, this combination produces a chemical reaction that generates toxic and carcinogenic vapors.

Further, the mixing of these two components must occur in relatively short times in order to avoid the drying reaction.

Moreover, mixing must occur so that no air (or gas) bubbles form inside, since said bubbles, once in place, reduce the mechanical strength of the cement and can also cause contaminations and infections.

Secondly, the cement obtained from this combination must sometimes be introduced in cavities that are difficult to fill, with the risk of leaving residual regions without cement, with an obvious reduction in mechanical strength.

Currently, all the devices used to mix these components and distribute the cement in the affected region have a common structure and operating principle.

Basically, these devices comprise a container in which the two components whose combination will produce the cement are introduced by means of various methods.

A piston or other variously shaped element, connected to the outside and closed by means of sealing gaskets, is arranged inside said container.

A more or less high vacuum can be produced inside the container by using a conventional manual or electric suction pump.

Once almost all the air is eliminated (if suction is used), the rotary or pressing motion of said piston produces the mixing of the two components.

In this manner, the resulting toxic gas is not dispersed into the environment; moreover, fewer bubbles are present in the compound because mixing occurs almost in the absence of air.

Once this step has ended, the container is usually opened (although there are rather complicated and expensive "closed cycle" solutions) in order to remove the internal piston and collect the resulting cement paste.

This operation can be performed in several manners, including manual gathering of the cement paste, manipulating it so as to obtain the intended consistency and shape, and then inserting it in the bone cavity.

As an alternative, it is possible to pour the paste; if it is left liquid enough, directly into the bone cavity by means of the container itself.

Moreover, it is possible to insert a piston in the container, converting it into a syringe and making the cement paste flow out through a nozzle arranged at the base of the container into the bone cavity to be filled.

These devices in any case have various drawbacks in different respects.

One of these drawbacks is that one is normally forced to reopen the container, thus releasing into the environment the residual gas generated during the mixing of the two components.

Moreover, when the mixing element (for example a rotating spatula) that is present inside the container is extracted in order to draw the paste from the container, it carries away part of the paste that remains attached to it.

Further, using these devices requires several manual operations that are often difficult and time-consuming and are always rather laborious.

Another drawback is the complexity and size of the elements that compose these devices, which do not allow easy and immediately understandable use on the part of nursing staff and of the physician who must position and introduce the cement in the cavity.

The problem of the release of the gas produced by the combination of the two components of the cement is solved by the previously mentioned "closed cycle" devices, but these devices are very complicated, almost always bulky, and expensive.

The complexity of these devices does not allow their economically convenient disposable use, which is an increasingly required characteristic for surgical instruments in order to avoid the sterilization costs and the unavoidable possible residual risks of transmission of infectious diseases.

One solution that substantially solves the problems noted earlier is disclosed in Italian patent application No. PD 2002 A 284 filed by this same Applicant.

This application discloses a mixing and distribution device for multicomponent bone cement that comprises a box-like body that forms inside it a cylindrical receptacle that is closed in an upper region by a removable fluid-tight lid and inside which a mixing/distribution screw feeder is accommodated.

A piston is coaxially arranged between the screw feeder and the lid and is associated with movement means. Said piston forms, inside the receptacle, on opposite sides with respect to its ends, a venting chamber toward the lid and a mixing chamber toward the bottom of the receptacle.

The mixing chamber and the venting chamber are connected respectively to a channel for distributing bone cement, which is controlled by first valve means, such as a plug valve, and to a venting channel, which is controlled by second valve means, such as a second plug valve.

The venting channel and the distribution channel can be associated functionally with means for producing vacuum, such as a vacuum pump.

The screw feeder can be associated with rotation means, such as an electric motor, by way of fixing lugs.

The vacuum pump allows to draw air and gas from the receptacle during the various steps for mixing and distributing the cement.

With this device there is no leakage or escape of toxic gas during the mixing of the components of the cement or in the subsequent steps, since any gas is aspirated continuously. The mixture is thus obtained in a practically airless environment, thus avoiding the formation of bubbles of air or gas.

All the resulting cement is used, since the rotation and thrust of the screw feeder tend to propel it out until it is depleted.

The unused fraction of cement, once the valves have closed again, can be left inside the box-like body and can be eliminated together with the container without producing environmental contamination.

Although this device solves most of the problems of known types, it is not free from drawbacks.

An important characteristic of these devices is that it must be possible for the health worker who uses them to handle them with a single hand during distribution of the cement paste on the part to be fixed; the other hand is in fact often busy with other surgical instruments.

The described device instead cannot be handled easily with a single hand, and this makes it awkward to use.

US-A-5 842 786 discloses a device for mixing medical compositions having a combination of features as set forth in the pre-characterizing portion of the appended claim 1.

### Disclosure of the Invention

The aim of the present invention is to provide a mixing and distribution device for fixing paste, particularly for multicomponent bone cement, that solves the drawbacks noted in known types.

Within this aim, an object of the present invention is to provide a mixing and distribution device for fixing paste, particularly for multicomponent bone cement, that works without loss or leakage of the gas generated by the mixing of the components of the cement and allows to extract the air from the container in which mixing occurs.

Another object of the present invention is to provide a mixing and distribution device for fixing paste, particularly for multicomponent bone cement, that can be handled with a single hand during distribution of the fixing paste.

Another object of the present invention is to provide a mixing and distribution device for fixing paste, particularly for multicomponent bone cement, in which the cement can flow out in a controlled and precise manner.

Another object of the present invention is to provide a mixing and distribution device for fixing paste, particularly for multicomponent bone cement, in which the components are mixed without producing air bubbles.

Another object of the present invention is to provide a mixing and distribution device for fixing paste, particularly for multicomponent bone cement, that is simple and compact, easy to use, and has a limited number of essential components.

Another object of the present invention is to provide a mixing and distribution device for fixing paste, particularly for multicomponent bone cement, that can be used disposably.

Another object of the present invention is to provide a mixing and distribution device for fixing paste, particularly for multicomponent bone cement, that can be produced by way of known systems and technologies.

In accordance with the invention, there is provided a mixing and distribution device for fixing paste, particularly for multicomponent bone cement, as defined in the appended claims.

### Brief Description of the Drawings

Further characteristics and advantages of the present invention will become better apparent from the following detailed description of a preferred but not exclusive embodiment thereof, illustrated by way of nonlimiting example in the accompanying drawings, wherein:

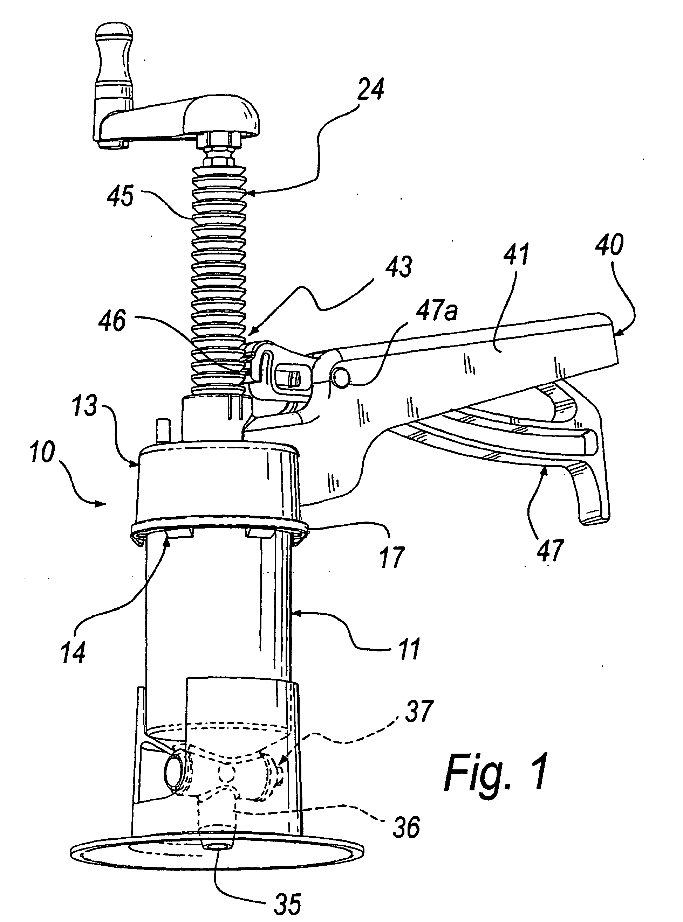

Figure 1 is a perspective view of the device according to the invention in the configuration for mixing the components of the multicomponent paste;

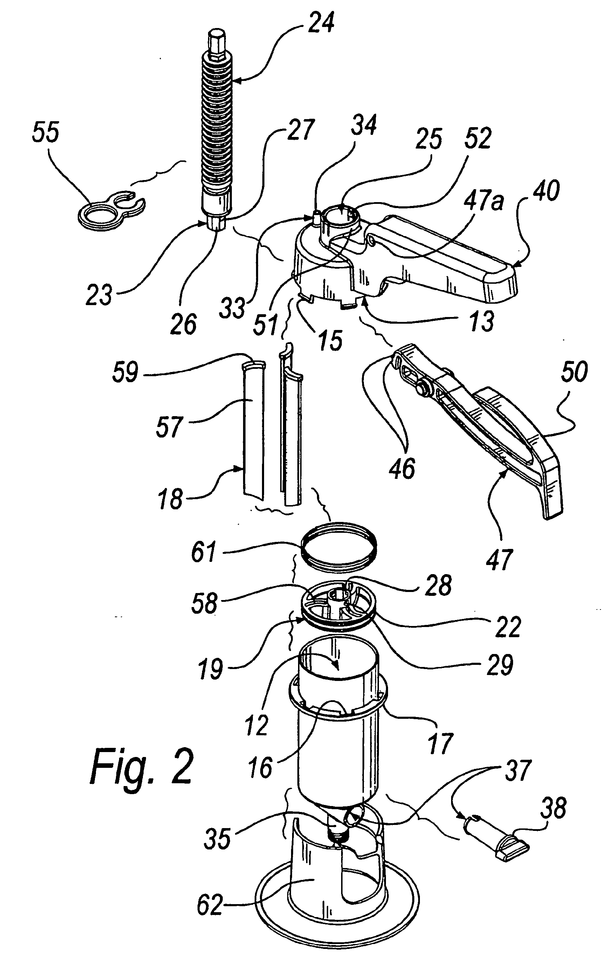

Figure 2 is an exploded view of the device according to the invention;

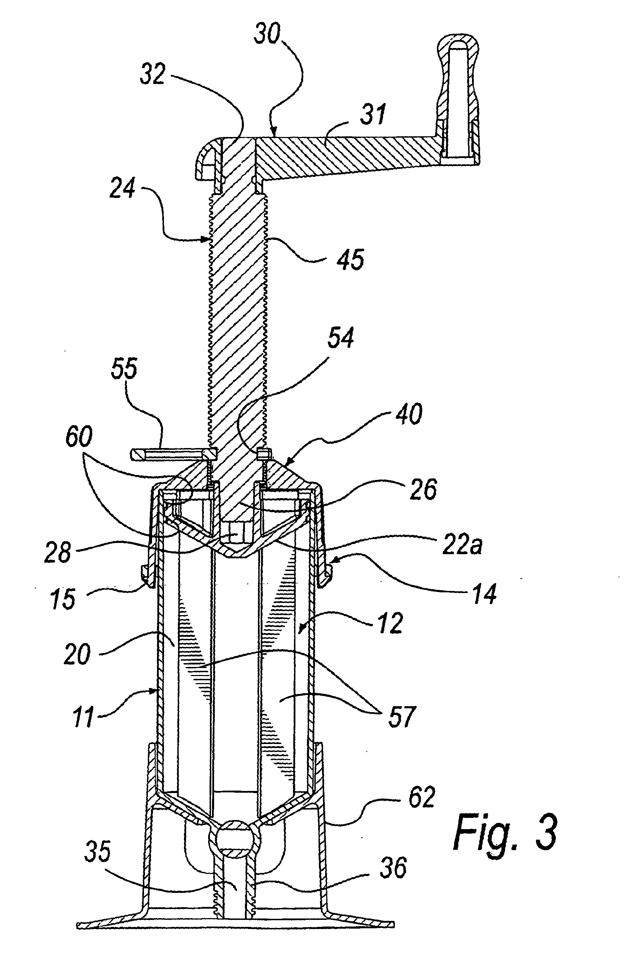

Figure 3 is a front sectional view of the device according to the invention;

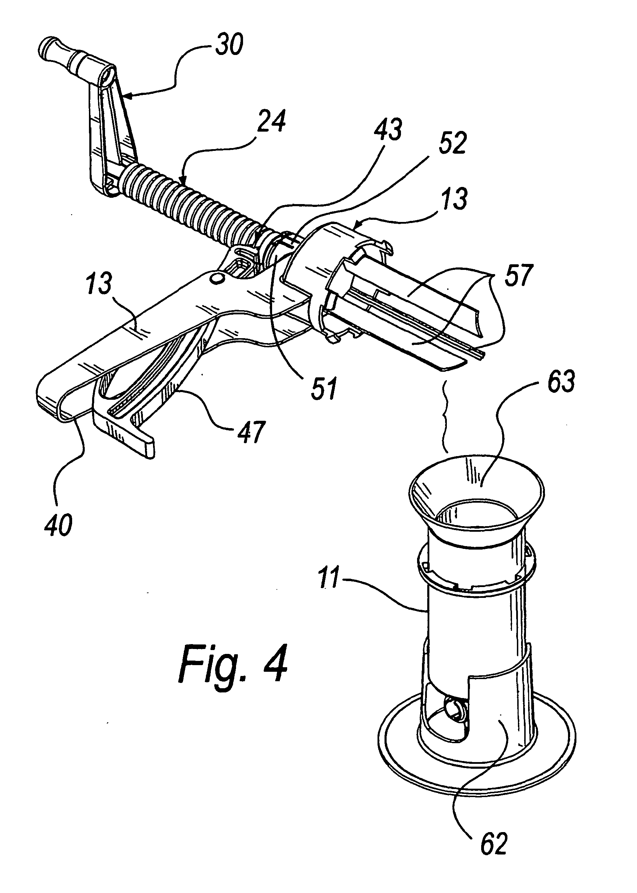

Figure 4 is a perspective view of the device according to the invention, divided into two separate preassembled parts;

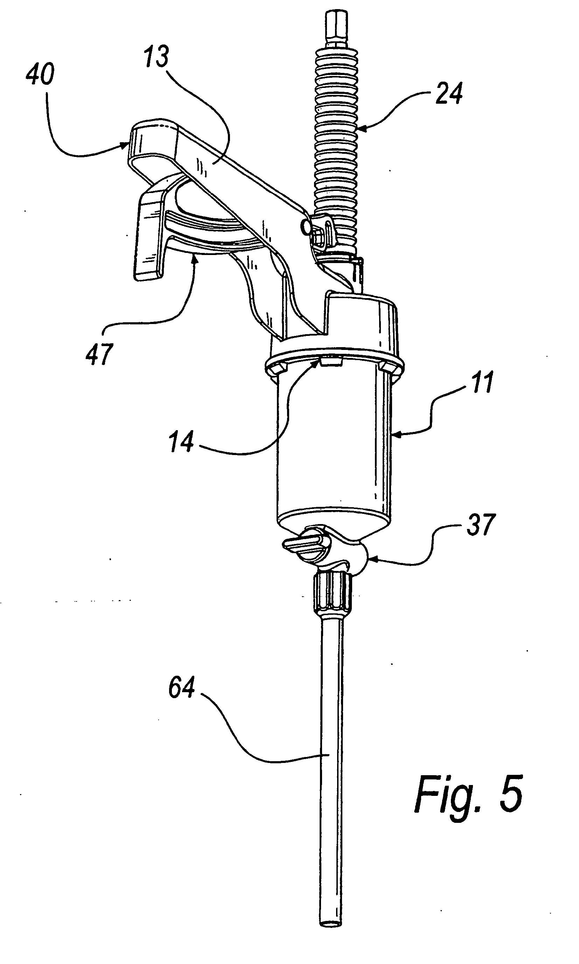

Figure 5 is a perspective view of the device according to the invention in the configuration for distributing the multicomponent paste.

### Ways of carrying out the Invention

With reference to the figures, a mixing and distribution device for fixing paste, particularly for multicomponent bone cement, according to the invention, is generally designated by the reference numeral 10.

The device 10 comprises a preferably cylindrical box-like body 11, which forms internally a cylindrical receptacle 12.

The cylindrical receptacle 12 is closed in an upper region by a detachable and substantially fluid-tight lid 13.

The lid 13 is internally cylindrical, with an inside diameter that has substantially the same dimensions as the outside diameter of the box-like body 11.

Externally, the lid 13 is preferably cylindrical (but it can also be for example polygonal).

Means 14 for detachable fixing to the box-like body 11 protrude from the rim of the lid 13; the fixing means 14 consist of teeth 15 of the snap-fit type, which enter by elastic deformation corresponding locking cavities 16 formed through a perimetric annular tab 17 that protrudes laterally from the cylindrical body 11.

Means 18 for mixing the components of the fixing paste, described hereinafter, are accommodated inside the cylindrical receptacle 12.

The mixing means 18 are rigidly coupled to a piston 19, which forms, inside the receptacle 12, a venting chamber (not shown in the figures, since the piston 19 is shown at the beginning of its stroke) toward the lid 13 and a mixing chamber 21 toward the bottom of the receptacle 12.

The volume of the two chambers can obviously vary according to the axial position occupied by the piston 19 inside the receptacle 12, as will become better apparent hereinafter.

The piston 19 has a first cylindrical portion 22, whose outside diameter is substantially equal to (at most slightly smaller than) the diameter of the receptacle 12, and a second portion 22a, which has a conical shape toward the bottom of the receptacle 12.

In an alternative embodiment, not shown in the figures, the vertex region of the second conical portion 22a has a coaxial protrusion that is substantially elongated downward.

A rotation pivot 24 is locked coaxially on the piston 19 by way of corresponding locking means 23 and is arranged so that it can slide through a corresponding substantially fluid-tight hole 25 formed in the lid 13.

The locking means 23 comprise a first shank 26, which preferably has a hexagonal cross-section and is formed at one end of the pivot 24; two studs 27 protrude on opposite sides from its lateral surface.

A seat 28 shaped complementarily to the first shank 26 is formed axially on the piston 19, and two openings 29 complementary to the two studs 27 are formed on its side walls; mutual coupling occurs by elastic deformation of the walls of the seat 28.

The rotation pivot 24 can be coupled to corresponding rotation means 30, which can be actuated by a user during the mixing of the multicomponent paste.

The rotation means 30 are constituted for example by a crank 31, which is detachably coupled to the free end of the pivot 24.

The free end of the pivot 24 is constituted by a second shank 32, which preferably has a hexagonal cross-section.

As an alternative, the means for rotating the pivot 24 can be constituted for example by an electric motor or, as an alternative, by a pneumatic motor (both of which are not shown in the figures), which are present in operating rooms (electric power supplies and pneumatic power supplies are in fact both present in operating rooms) when the device is used; a chuck for coupling to the second shank 32 is associable with said motors.

The venting chamber is connected to a venting channel 33, which can be functionally associated with suction means (not shown in the figures), such as for example a vacuum pump.

The venting channel 33 is constituted for example by a tube 34 that protrudes from the upper portion of the lid 13.

The mixing chamber 21 is connected to a channel 35 for distributing the bone cement toward the outside of the box-like container 11.

The distribution channel 35 protrudes from the bottom of the receptacle 12, which has a conical shape, by means of a nozzle 36 controlled by valve means 37, such as for example a plug valve 38.

The device 10 further comprises a handgrip 40 for one hand of a user during the distribution of the multicomponent paste.

The handgrip is constituted for example by a pistol grip 41, which is rigidly coupled to the lid 13.

In this embodiment of the invention, the pistol grip 41 is monolithic with the lid 13, but in alternative embodiments it is possible to use handgrips that are detachable with respect to the lid 13 (or can be optionally detached with respect to the box-like body 11).

Means 43 for producing the translational motion of the rotation pivot 24 (with the piston 19 rigidly coupled thereto), toward the bottom of the receptacle 12 are associated with the handgrip 40.

The translational motion means 43 are constituted, in this embodiment, by a ratchet-type mechanism, which is constituted by a plurality of annular bulges 45 that have a sawtooth transverse profile (in which the inclination of the teeth converges toward the lid 13) and are engaged by two complementarily shaped teeth 46 arranged monolithically on the first end of a first-class (centrally-pivoted) lever 47.

Elastic return means 49 that act between the lever 47 and the pistol grip 41 are provided between the fulcrum and the second end of the lever 47.

In particular, the elastic return means 49 are constituted by an elastically deformable leaf-spring element 50, which cantilevers out from the second end of the lever 47 toward said first end and abuts against the bottom of the pistol grip 41.

A collar 51 is provided on the lid 13, and the fluid-tight hole 25 is formed coaxially to the collar.

Tabs 52 for preventing reversibility of the translational motion (toward the bottom of the container 11) of the pivot 24 are provided on the collar 51; the tabs 52 are elastically deformable and are provided with teeth that are shaped complementarily with respect to the annular bulges 45 of the pivot 24, so as to prevent the accidental upward motion of the pivot 24.

A retention element 55 is associated with the pivot 24 and has a C-shaped part designed to be coupled reversibly, by elastic deformation, to an annular groove 54 that is formed on the pivot 24 at the beginning of the annular bulges 45 and is directed toward the first shank 26.

The retention element 55 is coupled to the annular groove 54 only during the mixing of the multicomponent paste.

Proximate to the first shank 26 there is also an annular pocket (not shown in the figures) for accommodating a corresponding sealing gasket.

The mixing means 18 are constituted for example by mixing blades 57, which are predominantly longitudinally elongated and are arranged so as to slide inside complementary slots 58 formed in the piston 19.

The length of the mixing blades 57 is equal to the internal length of the receptacle 12, and their transverse profile is curved in this embodiment, but other profiles can be used, also depending on the type of multicomponent paste to be mixed; the thickness of the mixing blades 57 is substantially constant, but in alternative embodiments it is also possible to use blades that have a variable thickness, for example a thickness that increases from the side of the container 11 toward the pusher piston 24.

The end of the mixing blades 57 that is directed toward the lid 13 has a head 59 that is wider than the complementary slots 58.

The coupling of the mixing blades 57 to the slots 58 is provided with small passages (not shown in the figures) for the outflow of the air and gas from the mixing chamber 21 to the venting chamber.

Circumferential pockets 60 for accommodating corresponding scraper rings 61 for the side wall of the receptacle 12, or alternatively sealing gaskets (not shown in the figures), are provided on the first cylindrical portion 22 of the piston 19.

Advantageously, the device comprises accessories, such as a stand 62 in which it is possible to insert the box-like container 11, so that it is directed downward, in order to mix the multicomponent paste, and a collar-funnel 63 to be arranged in abutment against the annular tab 17 and is useful during the pouring of the components of the multicomponent paste.

Moreover, the present invention comprises an extension nozzle 64 to be fixed to the nozzle 36, of the syringe-needle type, so as to precisely direct the paste during distribution on the parts to be fixed.

The invention is preferably entirely made of plastics.

The operation of the device according to the invention is as follows.

The container 11 is positioned on the stand 62 and the collar-funnel 63 is fitted.

At this point, the components of the paste are poured, the collar-funnel 63 is removed, and the assembly constituted by the lid 13, with the pivot 24 at the beginning of its stroke with respect to the hole 25 and the piston 19 adjacent to the lid 13, is inserted.

In this step, the heads 59 of the mixing blades 57 abut against the piston 19.

The venting tube 34 is connected to a vacuum pump and operated.

By way of the rotation means (crank or motor), the pivot 24 is turned and consequently the piston 19 is turned together with the blades 57.

In this step, the locking element 55 is coupled to the annular groove 54, preventing the pivot 24 with the piston 19 from moving downward.

Once the mixing step produced by the rotation of the blades 57 has ended, the user detaches the locking element 55 and grips the pistol grip 41, beginning to press the lever 47 toward the bottom of the pistol grip 41.

This movement produces the downward advancement of the teeth 46, consequently pushing likewise downward the pivot 24 and therefore the piston 19.

By way of the leaf-spring element 50, the lever 47 is returned into position in order to allow to continue pushing the teeth 46 on the annular bulges 45, lowering the piston 19.

The fulcrum of the lever 47 is formed by a hinge that is constituted by a circular hole 47a, which is provided on the pistol grip 41 and in which there is a pivot (not shown in the figures) that is rigidly coupled to the lever.

In an alternative embodiment, the hole 47a has an oval shape, in which the corresponding major axis is substantially oriented along the extension of the pistol grip 41.

The piston 19 slides on the mixing blade 57 by way of the slots 58 and until it makes contact with the mixed paste.

At this point, the mixing chamber is a substantially airless and gas-free environment.

At this point, by again acting on the pistol grip, the formed paste is compressed, pushing it outward through the distribution channel 35 (the plug valve 38 has been opened beforehand).

In practice it has been found that the invention thus described solves the problems noted in known types of mixing and distribution device for multicomponent bone cement.

In particular, with the present invention there is no loss or leakage of toxic gas during the mixing of the components of the cement, since any gas is continuously aspirated during the mixing step.

The mixture is thus obtained in a practically airless environment, thus avoiding the formation of air or gas bubbles.

Moreover, it is noted that all the cement that is obtained can be used, since the thrust of the piston 19 tends to push it out until it is depleted (the piston is shaped complementarily to the bottom of the receptacle 12).

The device is further compact and simple to use and handle.

In particular, by gripping it with a single hand one is able to direct the nozzle in the regions where the paste is to be distributed and to operate the mechanisms for distributing said paste.

Advantageously, the invention is substantially composed of two parts: a part that is provided in an already-assembled condition and comprises the lid with the handgrip, the pivot and the piston with mixing blades, and a part that relates to the box-like container inside which the paste is mixed.

In view of its limited structural complexity and of the fact that it is entirely made of plastic material, the device can be produced at extremely competitive costs and therefore used disposably.

The invention thus conceived is susceptible of numerous modifications and variations, all of which are within the scope of the appended claims; all the details may further be replaced with other technically equivalent elements.

In practice, the materials employed, so long as they are compatible with the specific use, as well as the dimensions, may be any according to requirements and to the state of the art.

## Claims

1. A mixing and distribution device for fixing paste, particularly for multicomponent bone cement, comprising a box-like body (11) that forms internally a receptacle (12) that is closed in an upper region by a detachable and substantially fluid-tight lid (13), inside which means (18) for mixing the components of the fixing paste are accommodated, said mixing means (18) being coupled to a piston (19) that forms, inside said receptacle (12), a venting chamber toward said lid (13) and a mixing chamber (21) toward the bottom of said receptacle (12), said mixing chamber (21) being connected to a channel (35) for distributing bone cement, which is controlled by valve means (37), said venting chamber being instead connected to a venting channel (33), which can be functionally associated with suction means, said mixing means (18) being constituted by longitudinally elongated mixing blades (57) that are arranged so as to slide within complementary through slots (58) formed in said piston (19), a rotation pivot (24) being further locked coaxially on said piston (19) and being arranged so that it can slide through a corresponding fluid-tight hole (25) formed in said lid (13), said rotation pivot (24) being associable with corresponding rotation means (30) that can be operated by a user during the mixing of the multicomponent paste, said device (10) further comprising a handgrip (40) for a single hand of the user, with which means (43) for the translational motion of said rotation pivot (24) together with said piston (19) toward the bottom of said receptacle (12) are associated, said means being actuatable with the same hand associated with said handgrip (40), said venting and mixing chambers (21) being mutually connected for the passage of gaseous fluids, **characterized in that** mixing blades (57) have a length equal to the internal length of the receptacle (12) that is closed in the upper region by said lid (13), wherein said piston (19) slides in said receptacle (12) on said mixing blades (57), and said venting channel (33) is constituted by a tube (34) that protrudes from the upper portion of said lid (13).

2. The device according to one or more of the preceding claims, wherein said handgrip (40) is constituted by a pistol grip (41) that is rigidly coupled to said lid (13).

3. The device according to one or more of the preceding claims, wherein said means (43) for the translational motion of said rotation pivot (24) comprise a ratchet mechanism that is constituted by a plurality of annular bulges (45) that have a sawtooth transverse profile in which the teeth are inclined so as to converge toward said lid (13), two complementarily shaped teeth (46) engaging on said annular bulges (45) and being arranged monolithically on the first end of a first-class lever (47), elastic return means (49) being provided between the fulcrum and said second end of said lever (47) and acting between said lever (47) and said pistol grip (41).

4. The device according to claim 3, wherein said elastic return means (49) comprise a leaf-spring element (50), which is elastically deformable and cantilevers out from said second end of said lever (47) toward said first end.

5. The device according to claims 3 or 4, wherein a collar (51) is present on said lid (13), said fluid-tight hole (25) being formed coaxially to said collar, tabs (52) for preventing reversibility of the translational motion of said pivot (24) being provided on said collar (51), said tabs (52) being elastically deformable and being provided with teeth that are shaped complementarily to said annular bulges (45) of said pivot (24) so as to prevent said pivot from rising accidentally.

6. The device according to one or more of the preceding claims, wherein a retention element (55) is associated with said pivot (24) and has a C-shaped part designed to be coupled reversibly by elastic deformation with an annular slot (54) formed on said pivot (24) at the beginning of said annular bulges (45), said retention element (55) being coupled to said annular slot (54) only during the mixing of the multicomponent paste.

7. The device according to one or more of the preceding claims, wherein the end of said mixing blades (57) that is directed toward said lid (13) has a head (59) that is wider than the width of the complementary slots (58).

8. The device according to one or more of the preceding claims, wherein the coupling of said mixing blades (57) to said slots (58) has passages for the outflow of the air and gas from said mixing chamber (21) toward said venting chamber.

9. The device according to one or more of the preceding claims, wherein the length of said mixing blades (57) is substantially equal to the internal length of said receptacle (12).

10. The device according to claim 9, wherein means (14) for detachable fixing to said box-like body (11) protrude from the rim of said lid (13).

11. The device according to claim 10, wherein said fixing means (14) are constituted by teeth (15) of the snap-fit type, to be coupled by elastic deformation within corresponding locking cavities (16) formed through a perimetric tab (17) that protrudes laterally from said cylindrical body (11).

12. The device according to one or more of the preceding claims, wherein said piston (19) has a first cylindrical portion (22) and a second portion (22a) that has a conical shape toward the bottom of said receptacle (12).

13. The device according to claim 12, wherein the vertex region of said second conical portion (22a) has a coaxial protrusion that is substantially elongated downward.

14. The device according to claim 12 or 13, wherein circumferential pockets (60) are provided on said first cylindrical portion (22) of said piston (19) in order to accommodate corresponding scraper rings (61) for the side wall of said receptacle (12) or, as an alternative, sealing gaskets.

15. The device according to claim 12 or 13, wherein circumferential pockets (60) for accommodating corresponding sealing gaskets are formed on said first cylindrical portion (22) of said piston (19).

16. The device according to one or more of the preceding claims, wherein said locking means (23) comprise a first shank (26) formed at one end of the pivot (24), two studs (27) protruding from the lateral surface of said first shank (26) on opposite sides, a seat (28) being formed axially in said piston (19) and being shaped complementarily to said first shank (26), openings (29) that are complementary to said two studs (27) being provided on the side walls of said first shank, the mutual coupling of said studs (27) and said openings (29) occurring by elastic deformation of the walls of said seat (28).

17. The device according to one or more of the preceding claims, wherein the free end of said pivot (24) is constituted by a second shank (32).

18. The device according to claim 17, wherein said rotation means (30) are constituted by a crank (31) that is coupled detachably to said second shank (32).

19. The device according to claim 17, wherein said rotation means (30) are constituted by an electric motor or a pneumatic motor, a spindle for coupling to said second shank (32) being associable with said motors.

20. The device according to claim 16, wherein an annular pocket for accommodating a corresponding gasket is formed proximate to said first shank (26).

21. The device according to one or more of the preceding claims, wherein said distribution channel (35) protrudes from the bottom of said receptacle (12) by way of a nozzle (36) that is controlled by said valve means (37), which consist of a plug valve (38).

22. The device according to one or more of the preceding claims, wherein it comprises a stand (62) in which it is possible to insert said box-like body (11) temporarily, with its bottom directed downward, in order to mix the multicomponent paste.

23. The device according to one or more of the preceding claims, wherein it comprises a collar-funnel (63) to be arranged temporarily in abutment against said perimetric tab (17) during the pouring of the components of the multicomponent paste.

24. The device according to one or more of the preceding claims, wherein it comprises an extension nozzle (64) of the syringe-needle type, to be coupled to the nozzle (36) in order to precisely direct the paste during distribution on the parts to be fixed.

25. The device according to one or more of the preceding claims, wherein it is entirely made of plastic material.

26. The device according to one or more of the preceding claims, wherein the fulcrum of said lever (47) is formed by a hinge constituted by a hole (47a), which is formed in said pistol grip (41) and in which there is a pivot that is rigidly coupled to said lever (47), said hole (47a) having an oval shape.

27. The device according to claim 26, wherein the major axis of said oval hole (47a) is substantially oriented along the extension of said pistol grip (41).

## Patentansprüche

1. Eine Misch- und Verteilvorrichtung für Fixierpaste, insbesondere für Mehrkomponenten-Knochenzement, die einen kastenähnlichen Körper (11) umfasst, welcher innen einen Behälter (12) bildet, der in einem oberen Bereich durch einen abnehmbaren und im Wesentlichen fluiddichten Deckel (13) verschlossen ist, in dem Mittel (18) zum Mischen der Komponenten der Fixierpaste untergebracht sind, wobei die Mischmittel (18) mit einem Kolben (19) gekoppelt sind, der innerhalb des Behälters (12) eine Lüftungskammer zum Deckel (13) hin und eine Mischkammer (21) zum Boden des Behälters (12) hin bildet, wobei die Mischkammer (21) mit einem Kanal (35) zur Verteilung von Knochenzement verbunden ist, der von VentilmitteIn (37) gesteuert wird, wobei die Lüftungskammer stattdessen mit einem Lüftungskanal (33) verbunden ist, der funktionell mit SaugmitteIn verknüpft werden kann, wobei die Mischmittel (18) durch in Längsrichtung gestreckten MischflügeIn (57) bestehen, die so angeordnet sind, um in komplementären durchgehenden Schlitzen (58) zu gleiten, die in dem Kolben (19) geformt sind, wobei weiter ein Drehzapfen (24) koaxial auf dem Kolben (19) verriegelt ist und so angeordnet ist, dass er durch ein entsprechendes im Deckel (13) geformtes fluiddichtes Loch (25) gleiten kann, wobei der Drehzapfen (24) mit entsprechenden Drehmitteln (30) verknüpft werden kann, die von einem Benutzer beim Mischen der Mehrkomponenten-Paste betätigt werden können, wobei die Vorrichtung (10) weiter einen Handgriff (40) für eine einzige Hand des Benutzers umfasst, mit welchem Mittel (43) für die translatorische Bewegung des Drehzapfens (24) gemeinsam mit dem Kolben (19) zum Boden des Behälters (12) hin verknüpft sind, wobei die Mittel mit derselben Hand bedient werden können, die den Handgriff (40) ergreift, wobei die Lüftungs- und Mischkammern (21) miteinander für das Durchströmen gasförmiger Fluide verbunden sind, **dadurch gekennzeichnet, dass** die Mischflügel (57) eine Länge gleich mit der Innenlänge des Behälters (12) haben, der im oberen Bereich von dem Deckel (13) verschlossen ist, wobei der Kolben (19) in dem Behälter (12) auf den Mischflügeln (57) gleitet und wobei der Lüftungskanal (33) aus einem Rohr (34) besteht, das aus dem oberen Abschnitt des Deckels (13) herausragt.

2. Die Vorrichtung gemäß einem oder mehreren der obigen Ansprüche, wobei der Handgriff (40) aus einem Auslösehandgriff (41) besteht, welcher starr mit dem Deckel (13) gekoppelt ist.

3. Die Vorrichtung gemäß einem oder mehreren der obigen Ansprüche, wobei die Mittel (43) für die translatorische Bewegung des Drehzapfens (24) einen Klinkenmechanismus umfassen, welcher aus einer Vielzahl ringförmiger Wulste (45) besteht, die ein transversales Sägezahnprofil haben, in dem die Zähne so geneigt sind, dass sie zum Deckel (13) hin zusammenlaufen, wobei zwei komplementär geformte Zähne (46) in die ringförmigen Wulste (45) eingreifen und monolithisch am ersten Ende eines zweiarmigen Hebels (47) angeordnet sind, wobei elastische Rückholmittel (49) zwischen dem Drehpunkt und dem zweiten Ende des Hebels (47) bereitgestellt sind und zwischen dem Hebel (47) und dem Auslösehandgriff (41) wirken.

4. Die Vorrichtung gemäß Anspruch 3, wobei die elastischen Rückholmittel (49) ein Blattfederelement (50) umfassen, das elastisch verformbar ist und vom zweiten Ende des Hebels (47) zum ersten Ende hin frei schwebt.

5. Die Vorrichtung gemäß Anspruch 3 oder 4, wobei eine Manschette (51) auf dem Deckel (13) vorhanden ist, wobei das fluiddichte Loch (25) koaxial zu der Manschette geformt ist, wobei Laschen (52) zum Verhindern einer Umkehrbarkeit der translatorischen Bewegung des Drehzapfens (24) an der Manschette (51) angebracht sind, wobei die Laschen (52) elastisch verformbar und mit Zähnen ausgestattet sind, welche komplementär zu den ringförmigen Wulsten (45) des Drehzapfens (24) geformt sind, um das Drehzapfen daran zu hindern, sich zufällig zu heben.

6. Die Vorrichtung gemäß einem oder mehreren der obigen Ansprüche, wobei ein Rückhalte-Element (55) mit dem Drehzapfen (24) verknüpft ist und einen C-förmigen Teil hat, der ausgebildet ist, durch elastische Verformung reversibel mit einem ringförmigen Schlitz (54) gekoppelt zu werden, der am Drehzapfen (24) am Anfang der ringförmigen Wulste (45) geformt ist, wobei das Rückhalte-Element (55) nur während des Mischens der Mehrkomponenten-Paste mit dem ringförmigen Schlitz (54) gekoppelt ist.

7. Die Vorrichtung gemäß einem oder mehreren der obigen Ansprüche, wobei das Ende der Mischflügel (57), das zum Deckel (13) hin ausgerichtet ist, einen Kopf (59) hat, der breiter ist als die Breite der komplementären Schlitze (58).

8. Die Vorrichtung gemäß einem oder mehreren der obigen Ansprüche, wobei die Kopplung der Mischflügel (57) mit den Schlitzen (58) Durchgänge für das Ausströmen der Luft und des Gases von der Mischkammer (21) zur Lüftungskammer hin hat.

9. Die Vorrichtung gemäß einem oder mehreren der obigen Ansprüche, wobei die Länge der Mischflügel (57) im Wesentlichen gleich der Innenlänge des Behälters (12) ist.

10. Die Vorrichtung gemäß Anspruch 9, wobei Mittel (14) zur lösbaren Befestigung am kastenähnlichen Körper (11) vom Rand des Deckels (13) abstehen.

11. Die Vorrichtung gemäß Anspruch 10, wobei die Befestigungsmittel (14) aus Zähnen (15) des Schnappverschluss-Typs bestehen, um durch elastische Verformung innerhalb von entsprechenden Verriegelungs-Vertiefungen (16)gekoppelt zu werden, die durch eine perimetrische Lasche (17) geformt sind, welche seitlich aus dem zylindrischen Körper (11) herausragt.

12. Die Vorrichtung gemäß einem oder mehreren der obigen Ansprüche, wobei der Kolben (19) einen ersten zylindrischen Abschnitt (22) und einen zweiten Abschnitt (22a) hat, der zum Boden des Behälters (12) hin eine Kegelform hat.

13. Die Vorrichtung gemäß Anspruch 12, wobei der Scheitelpunkt-Bereich des zweiten Kegelabschnitts (22a) einen koaxialen Vorsprung hat, der nach unten hin im Wesentlichen lang gestreckt ist.

14. Die Vorrichtung gemäß Anspruch 12 oder 13, wobei umlaufende Taschen (60) am ersten zylindrischen Abschnitt (22) des Kolbens (19) angebracht sind, zur Aufnahme von entsprechenden Abstreifringen (61) für die Seitenwand des Behälters (12), oder alternativ von Dichtringen.

15. Die Vorrichtung gemäß Anspruch 12 oder 13, wobei umlaufende Taschen (60) zur Aufnahme entsprechender Dichtringe an dem ersten zylindrischen Abschnitt (22) des Kolbens (19) geformt sind.

16. Die Vorrichtung gemäß einem oder mehreren der obigen Ansprüche, wobei die Verriegelungsmittel (23) einen ersten Schaft (26) umfassen, der an einem Ende des Drehzapfens (24) geformt ist, wobei zwei Stifte (27) auf gegenüberliegenden Seiten aus der seitlichen Oberfläche des ersten Schafts (26) heraus ragen, wobei ein Sitz (28) axial im Kolben (19) geformt und komplementär zu dem ersten Schaft (26) geformt ist, wobei Öffnungen (29), die komplementär zu den beiden Stiften (27) sind, an den Seitenwänden des ersten Schafts angebracht sind, wobei die Kopplung der Stifte (27) und der Öffnungen (29) miteinander durch elastische Verformung der Wände des Sitzes (28) stattfindet.

17. Die Vorrichtung gemäß einem oder mehreren der obigen Ansprüche, wobei das freie Ende des Drehzapfens (24) aus einem zweiten Schaft (32) besteht.

18. Die Vorrichtung gemäß Anspruch 17, worin die Drehmittel (30) aus einer Kurbel (31) bestehen, die lösbar mit dem zweiten Schaft (32) verbunden ist.

19. Die Vorrichtung gemäß Anspruch 17, worin die Drehmittel (30) aus einem Elektromotor oder einem pneumatischen Motor bestehen, wobei eine Spindel zur Kopplung mit dem zweiten Schaft (32) mit den Motoren verknüpft werden kann.

20. Die Vorrichtung gemäß Anspruch 16, worin eine ringförmige Tasche zur Aufnahme eines entsprechenden Dichtrings nahe dem ersten Schaft (26) geformt ist.

21. Die Vorrichtung gemäß einem oder mehreren der obigen Ansprüche, worin der Verteilungskanal (35) aus dem Boden des Behälters (12) durch eine Düse (36) herausragt, die durch die Ventilmittel (37) gesteuert wird, welche aus einem Stopfenventil (38) bestehen.

22. Die Vorrichtung gemäß einem oder mehreren der obigen Ansprüche, worin sie einen Ständer (62) umfasst, in den der kastenähnliche Körper (11) vorübergehend mit seiner Unterseite nach unten zum Mischen der Mehrkomponenten-Paste eingesetzt werden kann.

23. Die Vorrichtung gemäß einem oder mehreren der obigen Ansprüche, worin sie einen Kragentrichter (63) umfasst, der vorübergehend während des Gießens der Komponenten der Mehrkomponenten-Paste gegen die perimetrische Lasche (17) anstoßend anzuordnen ist.

24. Die Vorrichtung gemäß einem oder mehreren der obigen Ansprüche, worin sie eine Erweiterungsdüse (64) vom Spritzennadel-Typ umfasst, um mit der Düse (36) gekoppelt zu werden, um die Paste während der Verteilung der zu fixierenden Teile genau zu lenken.

25. Die Vorrichtung gemäß einem oder mehreren der obigen Ansprüche, worin sie vollständig aus Kunststoffmaterial besteht.

26. Die Vorrichtung gemäß einem oder mehreren der obigen Ansprüche, worin der Drehpunkt des Hebels (47) durch ein Scharnier geformt ist, das aus einem Loch (47a) besteht, welches in dem Auslösehandgriff (41) geformt ist und in dem sich ein Drehzapfen befindet, der starr mit dem Hebel (47) gekoppelt ist, wobei das Loch (47a) eine ovale Form hat.

27. Die Vorrichtung gemäß Anspruch 26, worin die Hauptachse des ovalen Lochs (47a) im Wesentlichen entlang der Verlängerung des Auslösehandgriffs (41) ausgerichtet ist.

## Revendications

1. Dispositif de mélange et de distribution pour pâte de fixation, en particulier pour osciment à composants multiples, comprenant un corps en forme de boîtier (11) qui forme intérieurement un réceptacle (12) qui est fermé dans une région supérieure par un couvercle (13) amovible sensiblement étanche aux fluides, à l'intérieur duquel sont logés des moyens (18) pour mélanger les composants de la pâte de fixation, lesdits moyens mélangeurs (18) étant couplés à un piston (19) qui forme, à l'intérieur dudit réceptacle (12), une chambre de ventilation vers ledit couvercle (13) et une chambre de mélange (21) vers le fond dudit réceptacle (12), ladite chambre de mélange (21) étant reliée à un canal (35) pour la distribution de l'os-ciment, qui est commandée par des moyens clapet (37), ladite chambre de ventilation étant en revanche reliée à un canal de ventilation (33) qui peut être associé de façon fonctionnelle à des moyens de succion, lesdits moyens mélangeurs (18) étant constitués par des lames de mélange (57) allongées longitudinalement qui sont agencées de sorte à coulisser à l'intérieur de fentes traversantes (58) complémentaires formées dans ledit piston (19), un pivot de rotation (24) étant en outre verrouillé coaxialement sur ledit piston (19) et étant agencé de sorte à pouvoir coulisser à travers un trou (25) étanche aux fluides correspondant formé dans ledit couvercle (13), ledit pivot de rotation (24) étant associable à des moyens de rotation (30) correspondants qui peuvent être actionnés par un utilisateur pendant le mélange de la pâte à composants multiples, ledit dispositif (10) comprenant en outre une poignée (40) pour une main de l'utilisateur, à laquelle sont associés des moyens (43) pour le déplacement translationnel dudit pivot de rotation (24) avec ledit piston (19) vers le fond dudit réceptacle (12), lesdits moyens étant actionnables par la même main associée à ladite poignée (40), lesdites chambres de ventilation et de mélange (21) étant mutuellement reliées pour le passage de fluides gazeux, **caractérisé en ce que** les lames de mélange (57) présentent une longueur égale à la longueur interne du réceptacle (12) qui est fermé dans la région supérieure par ledit couvercle (13), dans lequel ledit piston (19) coulisse dans ledit réceptacle (12) sur lesdites lames de mélange (57), et ledit canal de ventilation (33) est constitué par un tube (34) qui fait saillie depuis la partie supérieure dudit couvercle (13).

2. Dispositif selon une ou plusieurs des revendications précédentes, dans lequel ladite poignée (40) est constituée par une poignée (41) qui est rigidement couplée audit couvercle (13).

3. Dispositif selon une ou plusieurs des revendications précédentes, dans lequel lesdits moyens (43) pour le déplacement translationnel dudit pivot de rotation (24) comprennent un mécanisme à rochet qui est constitué par une pluralité de saillies annulaires (45) qui présentent un profil transversal en dents de scie dans lequel les dents sont inclinées de sorte à converger vers ledit couvercle (13), deux dents (46) conformées de façon complémentaire venant en prise sur lesdites saillies annulaires (45) et étant agencées de façon monolithique sur 1a première extrémité d'un levier du premier genre (47), des moyens de retour élastiques (49) étant prévus entre le point d'appui et ladite deuxième extrémité dudit levier (47) et agissant entre ledit levier (47) et ladite poignée (41).

4. Dispositif selon la revendication 3, dans lequel lesdits moyens de retour élastiques (49) comprennent un élément à ressort à lame (50) qui est élastiquement déformable et en porte-à-faux depuis ladite deuxième extrémité dudit levier (47) vers ladite première extrémité.

5. Dispositif selon les revendications 3 ou 4, dans lequel un collier (51) est présent sur ledit couvercle (13), ledit trou étanche aux fluides (25) étant formé coaxialement audit collier, des languettes (52) pour empêcher la réversibilité du déplacement translationnel dudit pivot (24) étant prévu(e)s sur ledit collier (51), lesdites languettes (52) étant élastiquement déformables et étant munies de dents qui sont conformées de façon complémentaire auxdites saillies annulaires (45) dudit pivot (24) de sorte à empêcher ledit pivot de se lever accidentellement.

6. Dispositif selon une ou plusieurs des revendications précédentes, dans lequel un élément de retenue (55) est associé audit pivot (24) et présente une partie en forme de C destinée à être couplée de façon réversible par déformation élastique à une fente annulaire (54) formée sur ledit pivot (24) au niveau du début desdites saillies annulaires (45), ledit élément de retenue (55) étant couplé à ledit fente annulaire (54) seulement pendant le mélange de la pâte à composants multiples.

7. Dispositif selon une ou plusieurs des revendications précédentes, dans lequel l'extrémité desdites lames de mélange (57) qui est dirigée vers ledit couvercle (13) présente une tête (59) qui est plus large que la largeur des fentes complémentaires (58).

8. Dispositif selon une ou plusieurs des revendications précédentes, dans lequel le couplage desdites lames de mélanges (57) auxdites fentes (58) présente un passage pour la sortie de l'air et du gaz depuis ladite chambre de mélange (21) vers ladite chambre de ventilation.

9. Dispositif selon une ou plusieurs des revendications précédentes, dans lequel la longueur desdits lames de mélanges (57) est sensiblement égale à la longueur interne dudit réceptacle (12).

10. Dispositif selon la revendication 9, dans lequel des moyens (14) pour la fixation amovible audit corps en forme de boîtier (11) font saillie depuis le bord dudit couvercle (13).

11. Dispositif selon la revendication 10, dans lequel lesdits moyens de fixation (14) sont constitués par des dents (15) du type à déclic, à coupler par déformation élastique à l'intérieur de cavités de verrouillage (16) correspondantes formées à travers une languette périmétrique (17) qui fait saillie latéralement depuis ledit corps cylindrique (11)

12. Dispositif selon une ou plusieurs des revendications précédentes, dans lequel ledit piston (19) présente une première partie cylindrique (22) et une deuxième partie (22a) qui présente une forme conique vers le fond dudit réceptacle (12).

13. Dispositif selon la revendication 12, dans lequel la région de sommet de ladite deuxième partie conique (22a) présente une saillie coaxiale qui est sensiblement allongée vers le bas.

14. Dispositif selon la revendication 12 ou 13, dans lequel des poches circonférentielesl (60) sont prévues sur ladite première partie cylindrique (22) dudit piston (19) afin dé loger des anneaux racleurs (61) correspondants pour la paroi latérale dudit réceptacle (12) ou, en alternative, des joints d'étanchéité.

15. Dispositif selon la revendication 12 ou 13, dans lequel des poches circonférentielles (60) pour loger des joints d'étanchéité correspondants sont formées sur ladite première partie cylindrique (22) dudit piston (19).

16. Dispositif selon une ou plusieurs des revendications précédentes, dans lequel lesdits moyens de verrouillage (23) comprennent une première queue (26) formée à une extrémité du pivot (24), deux petits bossages (27) faisant saillie depuis la surface latérale de ladite première queue (26) sur des côtés opposés, un logement (28) étant formé axialement dans ledit piston (19) et étant conformé de façon complémentaire à ladite première queue (26), des ouvertures (29) qui sont complémentaires auxdits deux petits bossages (27) étant prévus sur les parois latérales de ladite première queue, le couplage mutuel desdits petits bossages (27) et desdites ouvertures (29) survenant par déformation élastique des parois dudit logement (28).

17. Dispositif selon une ou plusieurs des revendications précédentes, dans lequel l'extrémité libre dudit pivot (24) est constitué par une deuxième queue (32).

18. Dispositif selon la revendication 17, dans lequel lesdits moyens de rotation (30) sont constitués par une manivelle (31) qui est couplée de façon amovible à ladite deuxième queue (32).

19. Dispositif selon la revendication 17, dans lequel lesdits moyens de rotation (30) sont constitués par un moteur électrique ou un moteur pneumatique, un mandrin pour le couplage à ladite deuxième queue (32) étant associable auxdits moteurs.

20. Dispositif selon la revendication 16, dans lequel une poche annulaire pour le logement d'un joint correspondant est formée à proximité de ladite première queue (26).

21. Dispositif selon une ou plusieurs des revendications précédentes, dans lequel ledit canal de distribution (35) fait saillie depuis le fond dudit réceptacle (12) au moyen d'une buse (36) qui est commandée par lesdits moyens clapet (37) qui consistent en un robinet à boisseau (38).

22. Dispositif selon une ou plusieurs des revendications précédentes, dans lequel il comprend un piédestal (62) dans lequel il est possible d'insérer temporairement ledit corps en forme de boîtier (11), avec son fond dirigé vers le bas, afin de mélanger la pâte à composants multiples.

23. Dispositif selon une ou plusieurs des revendications précédentes, dans lequel il comprend un collier-entonnoir (63) à agencer temporairement en butée contre ladite languette périmétrique (17) durant là coulée des composants de la pâte à composants multiples.

24. Dispositif selon une ou plusieurs des revendications précédentes, dans lequel il comprend une buse d'extension (64) du type seringue à aiguille, à coupler à la buse (36) afin de diriger de façon précise la pâte lors de la distribution sur les parties à fixer.

25. Dispositif selon une ou plusieurs des revendications précédentes, dans lequel il est entièrement réalisé en matière plastique.

26. Dispositif selon une ou plusieurs des revendications précédentes, dans lequel le point d'appui dudit levier (47) est formé par une charnière constituée par un trou (47a) qui est formé dans ladite poignée (41) et dans lequel se trouve un pivot qui est rigidement couplé audit levier (47), ledit trou (47a) présentant une forme ovale.

27. Dispositif selon la revendication 26, dans lequel l'axe majeur dudit trou ovale (47a) est sensiblement orienté le long de l'extension de ladite poignée (41).
